(19) Europäisches Patentamt
European Patent Office
Office européen des brevets

(11) Veröffentlichungsnummer: **0 051 155**
**B1**

# (12) EUROPÄISCHE PATENTSCHRIFT

(45) Veröffentlichungstag der Patentschrift: **23.01.85**

(51) Int. Cl.⁴: **C 07 D 249/08**, A 01 N 43/64

(21) Anmeldenummer: **81107902.9**

(22) Anmeldetag: **05.10.81**

(54) **Verwendung von 2-Aryloxy-2-triazolylalkancarbonsäureamiden zur Bekämpfung unerwünschten Pflanzenwuchses und diese enthaltende Herbizide.**

(30) Priorität: **05.11.80 DE 3041702**

(43) Veröffentlichungstag der Anmeldung: **12.05.82 Patentblatt 82/19**

(45) Bekanntmachung des Hinweises auf die Patenterteilung: **23.01.85 Patentblatt 85/4**

(84) Benannte Vertragsstaaten: **AT BE CH DE FR GB IT LI LU NL SE**

(56) Entgegenhaltungen:
**EP-A-0 010 270**
**EP-A-0 010 298**
**EP-A-0 010 691**
**EP-A-0 013 873**
**DE-A-2 550 566**
**DE-A-2 720 654**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Husslein, Gerd, Dr., Herrenbergstrasse 2, D-6702 Bad Duerkheim (DE)**
Erfinder: **Ammermann, Eberhard, Dr., Sachsenstrasse 3, D-6700 Ludwigshafen (DE)**
Erfinder: **Hamprecht, Gerhard, Dr., Rote-Turm-Strasse 28, D-6940 Weinheim (DE)**
Erfinder: **Wuerzer, Bruno, Dr. Dipl.-Landwirt, Ruedigerstrasse 13, D-6701 Otterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 051 155 B1

**Beschreibung**

Die vorliegende Erfindung betrifft die Verwendung von 2-Aryloxy-2-triazolylalkancarbonsäureamiden zur Bekämpfung unerwünschten Pflanzenwuchses und diese enthaltende Herbizide.

Die Verwendung von Phenoxyalkancarbonsäure-Derivaten als Herbizide, z. B. dem Dimethylaminsalz der 2,4-Dichlorphenoxyessigsäure, ist bekannt (Wegler, R. — Chemie der Pflanzenschutz- und Schädlingsbekämpfungsmittel, Bd. 5 Herbizide, 1977, S. 180). Ihr besonderer Vorzug beruht auf der Bekämpfung breitblättriger Pflanzenarten unter weitgehender Schonung von Gräsern und grasartigen Kulturpflanzen.

Es ist ferner bekannt, Azolyl-alkancarbonsäurederivate als Fungizide zu verwenden (DE-A-2 720 654).

Es ist ferner bekannt, 1,2,4- Triazol-1-yl-Verbindungen als Fungizide zu verwenden (EP-A-10 298). Hierdurch wird die Verwendung der Verbindungen als Herbizide nicht nahegelegt.

Es ist weiterhin bekannt, Azolylphenylessigsäurederivate als Fungizide zu verwenden (EP-A-10 270). Diese Verbindungen zeigen keine oder nur eine sehr geringe herbizide Wirkung.

Es ist auch bekannt, substituierte Pyrazole als Herbizide zu verwenden (DE-A-2 550 566).

Es wurde nun gefunden, daß Verbindungen der allgemeinen Formel I

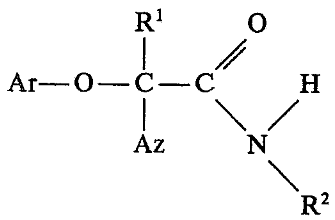

(I)

worin

Ar einen gegebenenfalls durch Halogen substituierten Naphthylrest oder einen gegebenenfalls durch Halogen, $C_1-C_4$-Alkyl, Phenyl, Benzyl, $CF_3$, Methoxy substituierten Phenylrest

$R^1$ Wasserstoff oder Methyl,

$R^2$ einen $C_1-C_{10}$-Alkyl-, $C_3-C_8$-Cycloalkyl-, Cyclopropylmethylrest, Allyl, Meth-Allyl, Crotyl, 2-Methyl-buten-2-yl-1, 2-Methylbuten-1-yl-3, Hexen-5-yl-1, 2-Methylbuten-1-yl-4, Propargyl, Butin-1-yl-3, Butin-2-yl-1, 3-Methyl-butin-1-yl-3 oder einen gegebenenfalls duch Halogen substituierten Aralkylrest mit 1 bis 2 C-Atomen im Alkylteil und 6 bis 10 C-Atomen im Arylteil,

Az einen gegebenenfalls durch Halogen oder Methyl substituierten 1-Triazolylrest bedeuten

und ihre Metallkomplexe herbizide und wachstumsregulierende Eigenschaften besitzen.

Dabei erstreckt sich die Wirkung sowohl auf breitblättrige wie auch auf grasartige Pflanzen.

Die Herstellung der Verbindungen der Formel I ist bekannt, und erfolgt durch die Umsetzung entsprechender 2-Aryloxy-2-halogenalkancarbonsäureamide mit Triazolen oder durch die Amidierung von 2-Aryloxy-2-triazolylalkancarbonsäureestern (DE-A-2 720 654) sowie durch die Umsetzung von 2-Aryloxy-2-triazolylalkancarbonsäuren — gegebenenfalls nach vorheriger Aktivierung mit beispielsweise Thionylchlorid oder Thionyldiazolen — mit Amminen (DE-A-2 846 038).

In der Formel I steht Ar vorzugsweise für einen durch Halogen, $C_1-C_4$-Alkyl, Trifluormethyl, einen Benzyl- oder Phenylrest einfach oder mehrfach substituierten Phenylrest, beispielsweise 2-Methyl-4-chlorphenyl, 2,4-Dichlorphenyl, 3,5-Dichlorphenyl, 2-Brom-4-chlorphenyl, 3,4-Dichlorphenyl, 2,4,5-Trichlorphenyl, 4-Fluorphenyl, 3-Trifluormethylphenyl, 3,5-Dimethylphenyl, 4-Benzylphenyl, o-Diphenyl, p-Diphenyl, 3-tert. Butylphenyl, 4-tert.-Butylphenyl; einen gegebenenfalls durch Halogen substitierten Naphthylrest, beispielsweise $\alpha$-Naphthyl-, $\beta$-Naphtyl; Verbindungen der Formel II worin Ar' = 3,5-Dichlorphenyl oder 3,5-Dimethylphenyl sind neu.

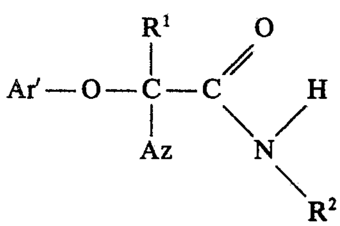

(II)

Beispiele für Verbindungen mit $R_2 = C_1-C_{10}$-Alkyl, oder $C_3$ bis $C_8$-Cycloalkylrest, sind: Methyl, Ethyl, n-Propyl, Isopropyl, Cyclopropyl, n-Butyl, Isobutyl, tert.-Butyl, Cyclobutyl, n-Pentyl, 2-Pentyl, 3-Pentyl, tert.-Amyl, Neopentyl, 2-Methyl-butyl, 3-Methyl-butyl, 3-Methyl-2-butyl, Cyclopentyl, n-Hexyl, 4-Methyl-2-pentyl, 2,3-Dimethylbutyl, 2-Methyl-1-pentyl, 2-Hexyl, 3-Hexyl, 3-Methyl-2-pentyl, 3-Methylpentyl, 4-Methylpentyl, 3-Methyl-3-pentyl, 4,4-Dimethylbutyl, Cyclohexyl, Heptyl, 2-Heptyl, 3-Heptyl, 4-Heptyl, Cycloheptyl, 1-Octyl, 3-Octyl, 4-Octyl, 5-Octyl, 5-Ethyl-2-heptyl, 2,6-Dimethyl-4-heptyl, 2,4-Dimethyl-3-pentyl, 3-Methyl-2-heptyl, Nonyl, 6-Ethyl-3-octyl, 2-Methyl-2-pentyl, 2,3-Dimethyl-2-butyl, 2-Methyl-2-hexyl, 3-Ethyl-3-pentyl, 3-Methyl-3-hexyl, 2,3-Dimethyl-2-butyl, 2,4-Dimethyl-2-pentyl, 2,2,3-Trimethyl-3-butyl, 2-Methyl-2-heptyl, 4-Methyl-4-heptyl, 2,4-Dimethyl-2-hexyl, 2-Methyl-2-octyl, oder Beispiele für einen durch Halogen substituierten Aralkylrest mit 1 bis 2 C-Atomen im Alkylteil und 6 bis 10 C-Atomen im Arylteil, sind Benzyl, p-Chlorbenzyl, α-Phenylethyl.

In einer besonders bevorzugten Klasse von Verbindungen der Formel I bedeutet Ar einen durch Halogen und/oder Methyl einfach oder mehrfach substituierten Phenylrest, $R^1$ Wasserstoff und $R^2$ einen verzweigten $C_3$ bis $C_7$-Alkylrest, beispielsweise Isopropyl, Isobutyl, tert. Butyl, tert. Amyl, 2,4-Dimethylpentyl-3.

Metallkomplexe sind die Komplexe mit Salzen von Metallen, z. B. Kupfer, Kobalt, Chrom. Die Metallkomplexe werden hergestellt durch Umsetzung der metallfreien Verbindungen mit den Metallsalzen, z. B. in einem organischen Lösungsmittel für die Verbindung.

Die Wirkstoffe enthalten ein asymmetrisches Kohlenstoffatom und bilden daher optisch aktive Isomere, die in üblicher Weise aus den bei der Herstellung entstehenden Racematen hergestellt werden können. Die vorliegende Erfindung betrifft sowohl die Racemate als auch die einzelnen optisch aktiven Isomeren.

Die Wirkstoffe werden beispielhaft durch nachstehende Tabelle erläutert.

| Nr. | Ar | Az | $R^1$ | $R^2$ | Fp. [°C] |
|---|---|---|---|---|---|
| 1 | 2,4-Dichlorphenyl | 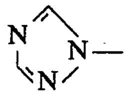 | H | $i\text{-}C_3H_7$ | 50– 53 |
| 2 | 2,4-Dichlorphenyl | desgl. | $CH_3$ | $i\text{-}C_3H_7$ | |
| 3 | 2,4-Dichlorphenyl | desgl. | H | tert. $C_4H_9$ | 107–108 |
| 4 | 2,4-Dichlorphenyl | desgl. | H | $-CH(i\text{-}C_3H_7)_2$ | 117–120 |
| 5 | 2,4-Dichlorphenyl | desgl. | H | 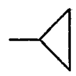 | 92– 96 |
| 6 | 2,4-Dichlorphenyl | desgl. | H | $-C(CH_3)_2C\equiv CH$ | 112–115 |
| 7 | 2,4-Dichlorphenyl | desgl. | H | $CH_2$— 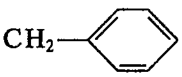 | 71– 75 |
| 8 | 4-Chlorphenyl | desgl. | H | tert. $C_4H_9$ | 176–180 |
| 9 | 2,4,5-Trichlorphenyl | desgl. | H | tert. $C_4H_9$ | 128–131 |
| 10 | 4-Fluorphenyl | desgl. | H | tert. $C_4H_9$ | 90– 94 |
| 11 | 4-Diphenyl | desgl. | H | tert. $C_4H_9$ | 114–118 |
| 12 | 2-Diphenyl | desgl. | H | tert. $C_4H_9$ | 142–146 |
| 13 | 4-Chlorphenyl | desgl. | H | $C(CH_3)_2C{=}CH$ | 74– 78 |
| 14 | 3,5-Dimethylphenyl | desgl. | H | tert. $C_4H_9$ | 131–133 |
| 15 | Phenyl | desgl. | H | tert. $C_4H_9$ | 101–103 |
| 16 | 2-Methyl-4-chlorphenyl | desgl. | H | tert. $C_4H_9$ | 119–122 |
| 17 | 4-tert.-Butylphenyl | desgl. | H | tert. $C_4H_9$ | 126–129 |
| 18 | 4-Benzylphenyl | desgl. | H | tert. $C_4H_9$ | 102–105 |
| 19 | α-Naphthyl | desgl. | H | tert. $C_4H_9$ | 110–114 |
| 20 | 3-Trifluormethylphenyl | desgl. | H | tert. $C_4H_9$ | 129–132 |
| 21 | 2,4-Dichlorphenyl | desgl. | H | $CH_2-CH(CH_3)_2$ | |
| 22 | 2,4-Dichlorphenyl | desgl. | H | tert. $C_4H_9$ $CuCl_2$-Komplex | 172–176 |
| 23 | 3-tert.-Butylphenyl | desgl. | H | tert. $C_4H_9$ | 97– 99 |
| 24 | 3,5-Dimethylphenyl | desgl. | H | $-C(CH_3)_2-C\equiv CH$ | 96– 98 |
| 25 | 3,5-Dimethylphenyl | desgl. | H | Benzyl | 97– 98 |
| 26 | 3,5-Dichlorphenyl | desgl. | H | tert. $C_4H_9$ | 104–106 |

Fortsetzung

| Nr. | Ar | Az | $R^1$ | $R^2$ | Fp. [°C] |
|---|---|---|---|---|---|
| 27 | 3,5-Dichlorphenyl | 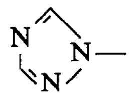 | H | Benzyl | 106–108 |
| 28 | 3,5-Dichlorphenyl | desgl. | H | $-C(CH_3)-C\equiv CH$ | |
| 29 | 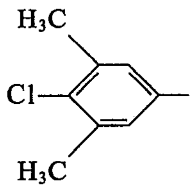 | desgl. | H | tert. $C_4H_9$ | 129–131 |
| 30 | desgl. | desgl. | H | 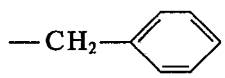 | 111–114 |
| 31 | 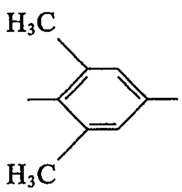 | desgl. | H | 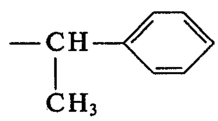 | 176–180 |
| 32 | desgl. | desgl. | H | 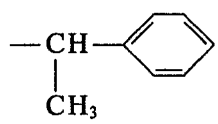 (opt.aktives Isomeres) | 111–114 |
| 33 | 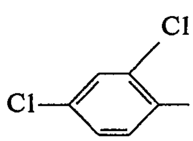 | desgl. | H | 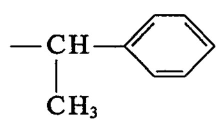 | 102–103 |
| 34 | desgl. | desgl. | H | 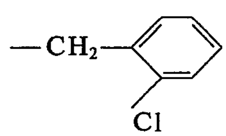 | 102–105 |
| 35 | 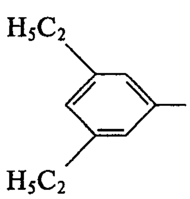 | desgl. | H | tert. $C_4H_9$ | 106–108 |
| 36 | 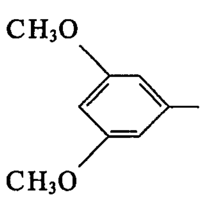 | desgl. | H | tert. $C_4H_9$ | 140–144 |
| 37 | 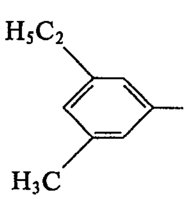 | desgl. | H | tert. $C_4H_9$ | 85– 89 |

Fortsetzung

| Nr. | Ar | Az | $R^1$ | $R^2$ | Fp. [°C] |
|---|---|---|---|---|---|
| 38 | 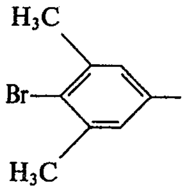 | 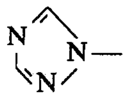 | H | tert. $C_4H_9$ | 107–112 |
| 39 | 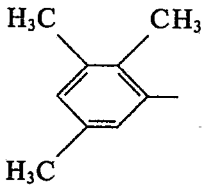 | desgl. | H | tert. $C_4H_9$ | 103–107 |
| 40 | 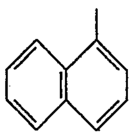 | desgl. | H | $—CH_2—CH(CH_3)_2$ | 105–107 |
| 41 | desgl. | desgl. | H | sek.-$C_4H_9$ | 128–130 |
| 42 | desgl. | desgl. | H | $—CH(CH_3)_2$ | 128–130 |
| 43 | desgl. | desgl. | H | 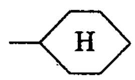 | 180–182 |
| 44 | 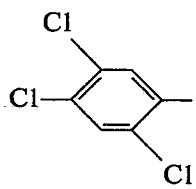 | desgl. | H | $—CH_2—$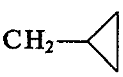 | 128–130 |
| 45 | desgl. | desgl. | H | sek.-$C_4H_9$ | 119–120 |
| 46 | desgl. | desgl. | H | $—CH(CH_3)_2$ | 118–119 |
| 47 | desgl. | desgl. | H | 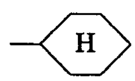 | 105–108 |
| 48 | 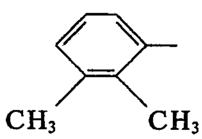 | desgl. | H | text.-$C_4H_9$ | 79– 80 |
| 49 | desgl. | desgl. | H | $—CH(CH_3)_2$ | 110–111 |
| 50 | desgl. | desgl. | H | 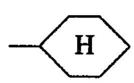 | 117–118 |
| 51 | desgl. | desgl. | H | $—CH_2—$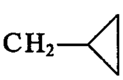 | |
| 52 | desgl. | desgl. | H | sek.-$C_4H_9$ | |

Die Wirkstoffe können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermiteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z. B. Xylol, Benzol), chlorierte Aromaten (z. B. Chlorbenzole), Paraffine (z. B. Edölfraktionen), Alkohole (z. B. Methanol, Butanol), Amine (z. B. Ethanolamin, Dimethylformamid) und Wasser; Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel wie nichtionogene und anionische Emulgatoren (z. B. Polyoxyethylen — Fettalkohol — Ether, Alkylsulfonate und Arylsulfonate) und Dispergiermittel, wie Lignin, Sulfitablaugen und Methylcellulose.

Zur Aktivierung der herbiziden Wirkung können Netz- und Haftmittel sowie nicht-phytotoxische Öle und Ölkonzentrate zugesetzt werden.

Die herbiziden Mittel enthalten im allgemeinen zwischen 0,1 und 95 Gew.-% Wirkstoff, vorzugsweise zwischen 0,5 und 90.

Die Aufwandmengen liegen je nach Zusammensetzung und Wachstumsstadien der Unkrautflora zwischen 0,1 und 15, vorzugsweise jedoch zwischen 0,2 und 5 kg Wirkstoff pro Hektar, wobei für eine totale Pflanzenvernichtung die höheren Aufwandmengen zu verwenden sind.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung 1 mit 10 Gewichtsteilen N-Methyl-alpha-pyrrolidon und erhält eine Lösung, die zur Anwendung in Form kleinster Tropfen geeignet ist.

II. Man vermischt 80 Gewichsteile der Verbindung 2 mit 20 Gewichtsteilen Cyclohexanon. Die Mischung kann in Form feiner Tropfen versprüht werden.

III. 10 Gewichtsteile der Verbindung 3 werden in einer Mischung gelöst, die aus 60 Gewichtsteilen Cyclohexanon, 20 Gewichtsteilen Isobutanol, 10 Gewichtsteilen des Anlagerungsproduktes von 40 Mol Ethylenoxid an 1 Mol Ricinusöl besteht.

IV. 3 Gewichtsteile der Verbindung des Beispiels 2 werden mit 97 Gewichsteilen feinteiligem Kaolin innig vermischt. Man erhält auf diese Weise ein Stäubemittel, das 3 Gew.-% des Wirkstoffs enthält.

V. 30 Gewichtsteile der Verbindung des Beispiels 3 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VI. 20 Teile der Verbindung 1 werden mit 2 Teilen Calciumsalz der Dodecylbenzolsulfonsäure, 8 Teilen Fettalkoholpolyglykolether, 2 Teilen Natriumsalz eines Phenolsulfonsäureharnstoff-formaldehyd-Kondensates und 68 Teilen eines paraffinischen Mineralöls innig vermischt. Man erhält eine stabile ölige Dispersion.

Die Wirkstoffe können allein, unter sich oder mit anderen Herbiziden sowie auch noch mit weiteren Pflanzenschutzmitteln gemischt und gemeinsam ausgebracht werden, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden.

Die Wirkung der Wirkstoffe auf das Wachstum von Pflanzen wird anhand von Gewächshausversuchen erläutert.

Als Kulturgefäße dienten Plastikblumentöpfe mit 300 $cm^3$ Inhalt und lehmigem Sand mit etwa 1,5% Humus als Substrat. Die Samen der Testpflanzen nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düse gespritzt. Bei dieser Applikationsmethode handelt es sich um eine Aufwandmenge entsprechend 3,0 kg Wirkstoff/ha. Nach dem Aufbringen der Mittel wurden die Gefäße leicht beregnet, um Keimung und Wachstum in Gang zu bringen. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Wirkstoffe beeinträchtigt wurde. Zum Zwecke der Nachauflaufbehandlung zog man die Testpflanzen je nach Wuchsform erst bis zu einer Wuchshöhe von 3 bis 10 cm an und behandelte sie danach. Zur Nachauflaufbehandlung wurden entweder direkt gesäte und in den gleichen Gefäßen aufgewachsene Pflanzen ausgewählt, oder sie wurden erst als

Keimpflanzen getrennt angezogen und einige Tage vor der Behandlung in die Versuchsgefäße verpflanzt. Die Aufwandmenge für die Nachauflaufbehandlung betrug 0,5 und 1,0 kg und in einigen Fällen 3,0 kg Wirkstoff/ha bezogen auf das Gesamtmolekül.

Als Vergleichsmittel dienten das Dimethylaminsalz der 2,4-Dichlorphenoxyessigsäure (A) mit derselben Dosis bezogen auf die Säure und das (2,4-Dichlorphenoxy)-(1,2,4-triazol-1-yl)-essigsäure-amid (B) (DE-A-2 720 654) mit der gleichen Aufwandmenge berechnet auf das Gesamtmolekül. Eine Abdekkung unterblieb bei der Nachauflaufbehandlung. Die Aufstellung der Versuche erfolgte im Gewächshaus, wobei für wärmeliebende Arten wärmere Bereiche (20 bis 30°C) und für solche gemäßigter Klimate 15 bis 25°C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Die Ergebnisse zeigen bei Nachauflaufanwendung im Gewächshaus die erwartete herbizide Wirkung des Vergleichsmittels A gegen breitblättrige unerwünschte Pflanzen mit einer geringen Schädigung von Gräsern und der grasartigen Kulturpflanze Gerste. Das Vergleichsmittel B hatte bei derselben Anwendungsmethode bei den meisten Testpflanzen keine oder im Einzelfall eine ganz geringe Aktivität. Der Wirkstoff Nr. 19 hatte im Gewächshaus bei Nachauflaufbehandlung dagegen eine sehr breite Wirkung gegen breitblättrige und grasartige Pflanzen. Dabei handelte es sich nicht nur um herbizid abtötende Effekte, sondern bei den Grasarten vorzugsweise um ein Zurückhalten des Wuchses (d. h. verminderte Wuchshöhe).

Bei diesen Versuchen zeigte ferner die Verbindung Nr. 21 im Gewächshaus bei Nachauflaufanwendung eine weitaus bessere herbizide Wirkung als das Vergleichsmittel B.

Bei Vor- und Nachauflaufanwendung im Gewächshaus zeigten die Wirkstoffe Nr. 15, 3, 5, 20 und 1 eine gute herbizide Wirkung.

In weiteren Gewächshausversuchen zeigten die Verbindungen Nr. 31, Nr. 29 und Nr. 26 bei Nachauflaufanwendung von 0,5 kg Wirkstoff/ha eine starke zunächst hemmende später zunehmende herbizide Wirkung auf breitblättrige wie auch grasartige Pflanzen.

Die wachstums- und bioreglierenden Eigenschaften der Wirkstoffe lassen sich in einem weiteren Gewächshausversuch mit Baumwolle demonstrieren. Bei dieser an sich mehrjährigen Kultur möchte man bei einjährigem Anbau das vegetative und generative Wachstum zum Ende der ersten Saison abgeschlossen haben. Ebenso ist es nachteilig, daß bereits entblätterte und zur Ernte vorgesehene Baumwollpflanzen wieder austreiben und sich neu begrünen. In einem Modellversuch mit blühenden und Kapseln ansetzenden Baumwollpflanzen konnten die Verbindungen Nr. 14, Nr. 31, Nr. 26 bei Blattbehandlung bei Aufwandmengen von 1,0 und 4,0 kg Wirkstoff/ha dieses unerwünsche Weiterwachsen der Baumwollpflanzen unterbinden oder stark eindämmen.

Die Applikation der Wirkstoffe bzw. der Mittel kann im Vorauflaufverfahren oder bei Nachauflaufanwendung, vorzugsweise bei Blattbehandlung erfolgen. Sind die Wirkstoffe bei Kulturpflanzen unverträglich, so können auch Ausbringungstechniken angewandt weden, bei welchen die herbiziden und wuchshemmenden Mittel mit Hilfe der Spritzgeräte so gespritzt werden, daß empfindliche Teile der Kulturpflanzen nicht getroffen weden, während die Wirkstoffe auf die Blätter darunter wachsender unerwünschter Pflanzen oder die unbedeckte Bodenfläche gelangen (post-directed, lay-bay).

Die Aufwandmengen an Wirkstoff oder aktiver Säure betragen je nach Jahreszeit und Wachstumsstadium er Zeilpflanzen 0,1 bis 15 kg/ha und mehr, wobei sich die höheren Dosen besonders zur totalen Bekämpfung von Vegetation eignen.

Zur Verbreiterung des Wirkungsspektrums und zur Erzielung synergistischer Effekte können die $\alpha$-Azolylalkancarbonsäureamide mit zahlreichen Vertretern anderer herbizider oder wachstumsregulierender Wirkstoffgruppen gmischt und gemeinsam ausgebracht werden. Beispielsweise kommen als Mischungspartner Diazine, Benzothiadiazinone, 2,6-Dinitroaniline, N-Phenylcarbamate, Thiolcarbamate, Halogencarbonsäure, Triazine, Amide, Harnstoffe, Diphenylether, Triazinone, Uracile, Benzofuranderivate, Cyclohexan-1,3-dionderivate und andere in Betracht.

Außerdem ist es nützlich, die Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Zur Aktivierung der hebiziden Wirkung können auch Netz- und Haftmittel sowie nicht-phytotoxische Öle und Ölkonzentrate zugesetzt werden.

**Patentansprüche für die Vertragsstaaten: BE, CH, DE, FR, GB, IT, LI, LU, NL, SE**

1. Verwendung einer Verbindung der Formel I

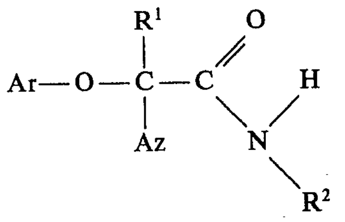

(I)

worin

Ar einen gegebenenfalls durch Halogen substituierten Naphthylrest oder einen gegebenenfalls durch Halogen, $C_1-C_4$-Alkyl, Phenyl, Benzyl, $CF_3$, Methoxy substituierten Phenylrest,
$R^1$ Wasserstoff oder Methyl,
$R^2$ einen $C_1-C_{10}$-Alkyl-, $C_3-C_8$-Cycloalkyl-, Cyclopropylmethhylrest, Allyl, Meth-allyl, Crotyl, 2-Methyl-buten-2-yl-1, 2-Methylbuten-1-yl-3, Hexen-5-yl-1, 2-Methylbuten-1-yl-4, Propargyl, Butin-1-yl-3, Butin-2-yl-1, 3-Methyl-butin-1-yl-3 oder einen gegebenenfalls duch Halogen substituierten Aralkylrest mit 1 bis 2 C-Atomen im Alkylteil und 6 bis 10 C-Atomen im Arylteil,
Az einen gegebenenfalls durch Halogen oder Methyl substituierten 1-Triazolylrest bedeuten oder ihren Metallkomplexen zur Bekämpfung unerwünschten Pflanzenwachstums.

2. Verbindung der Formel II

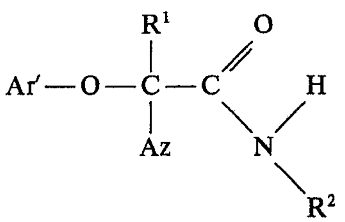

(II)

worin

Ar' einen 3,5-Dichlorphenyl- oder 3,5-Dimethylphenylrest,
$R^1$ Wasserstoff oder Methyl,
$R^2$ einen $C_1-C_{10}$-Alkyl-, $C_3-C_8$-Cycloalkyl-, Cyclopropylmethylrest, Allyl, Meth-allyl, Crotyl, 2-Methyl-buten-2-yl-1, 2-Methylbuten-1-yl-3, Hexen-5-yl-1, 2-Methylbuten-1-yl-4, Propargyl, Butin-1-yl-3, Butin-2-yl-1, 3-Methyl-butin-1-yl-3 oder einen gegebenenfalls durch Halogen substituierten Aralkylrest mit 1 bis 2 C-Atomen im Alkylteil und 6 bis 10 C-Atomen im Arylteil,
Az einen gegebenenfalls durch Halogen oder Methyl substituierten 1-Triazolylrest bedeuten oder ihre Metallkomplexe.

3. Verbindungen gemäß Anspruch 2, nämlich 2-(3,5-Dichlorphenoxy)-2-(1,2,4-triazolyl-1)-essigsäure-N-tertiärbutylamid, 2-(3,5-Dichlorphenoxy)-2-(1,2,4-triazolyl-1)-essigsäure-N-benzylamid.
4. Herbizid, enthaltend eine Verbindung gemäß Anspruch 2.
5. Herbizid, enthaltend eine Verbindung gemäß Anspruch 3.
6. Verwendung gemäß Anspruch 1, dadurch gekennzeichnet, daß man eine Verbindung gemäß Anspruch 2 verwendet.
7. Verwendung gemäß Anspruch 1, dadurch gekennzeichnet, daß man eine Verbindung gemäß Anspruch 3 verwendet.

**Patentansprüche für den Vertragsstaat: AT**

1. Verwendung einer Verbindung der Formel I

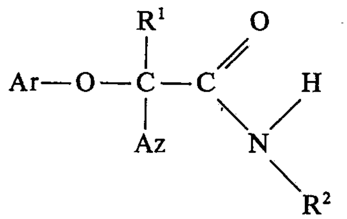

(I)

worin

Ar einen gegebenenfalls durch Halogen substituierten Naphthylrest oder einen gegebenenfalls durch Halogen, $C_1-C_4$-Alkyl, Phenyl, Benzyl, $CF_3$, Methoxy substituierten Phenylrest

$R^1$ Wasserstoff oder Methyl,

$R^2$ einen $C_1-C_{10}$-Alkyl-, $C_3-C_8$-Cycloalkyl-, Cyclopropylmethylrest, Allyl, Meth-allyl, Crotyl, 2-Methyl-buten-2-yl-1, 2-Methylbuten-1-yl-3, Hexen-5-yl-1, 2-Methylbuten-1-yl-4, Propargyl, Butin-1-yl-3, Butin-2-yl-1, 3-Methyl-butin-1-yl-3 oder einen gegebenenfalls durch Haligen substituierten Aralkylrest mit 1 bis 2 C-Atomen im Alylteil und 6 bis 10 C-Atomen im Arylteil,

Az einen gegebenenfalls durch Haligen oder Methyl substituierten 1-Triazolylrest bedeuten oder ihren Metallkomplexen zur Bekämpfung unerwünschten Pflanzenwachstums.

2. Herbizid, enthaltend eine Verbindung der Formel II

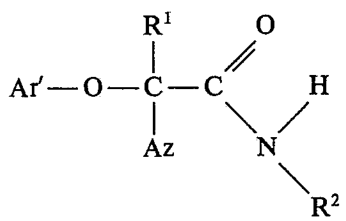

(II)

worin

Ar' einen 3,5-Dichlorphenyl oder 3,5-Dimethylphenylrest,

$R^1$ Wasserstoff oder Methyl,

$R^2$ einen $C_1-C_{10}$-Alkyl-, $C_3-C_8$-Cycloalkyl-, Cyclopropylmethylrest, Allyl, Meth-allyl, Crotyl, 2-Methyl-buten-2-yl-1, 2-Methylbuten-1-yl-3, Hexen-5-yl-1, 2-Methylbuten-1-yl-4, Propargyl, Butin-1-yl-3, Butin-2-yl-1, 3-Methyl-butin-1-yl-3 oder einen gegebenenfalls durch Halogen substituierten Aralkylrest mit 1 bis 2 C-Atomen im Alkylteil und 6 bis 10 C-Atomen im Arylteil,

Az einen gegebenenfalls durch Halogen oder Methyl substituierten 1-Triazolylrest bedeuten oder ihre Metallkomplexe.

3. Herbizid nach Anspruch 2, enthaltend 2-(3,5-Dichlorphenoxy)-2-(1,2,4-triazolyl-1)-essigsäure-N-tertiärbutylamid oder 2-(3,5-Dichlorphenoxy)-2-(1,2,4-triazolyl-1)-essigsäure-N-benzylamid.

4. Verwendung gemäß Anspruch 1, dadurch gekennzeichnet, daß man eine Verbindung gemäß Anspruch 2 verwendet.

5. Verwendung nach Anspruch 1, dadurch gekennzeichnet, daß man eine Verbindung gemäß Anspruch 3 verwendet.

**Claims for the Contrasting states: BE, CH, DE, FR, GB, IT, LI, LU, NL, SE**

1. The use of a compound of the formula I

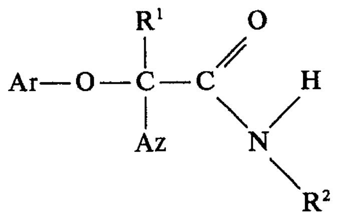

(I)

where

Ar is naphthyl optionally substituted by halogen or phenyl optionally substituted by halogen, alkyl of 1 to 4 carbon atoms, phenyl, benzyl, $CF_3$ or methoxy,
$R^1$ is hydrogen or methyl,
$R^2$ is alkyl of 1 to 10 carbon atoms, cycloalkyl of 3 to 8 carbon atoms, cyclopropylmethyl, allyl, methallyl, crotyl, 2-methylbut-2-en-1-yl, 2-methylbut-1-en-3-yl, hex-5-en-1-yl, 2-methylbut-1-en-4-yl, propargyl, but-1-yn-3-yl, but-2-yn-1-yl, 3-methylbut-1-yn-3-yl or an optionally halogen-sustituted aralkyl having 1 or 2 carbon atoms in the alkyl moiety and 6 to 10 carbon atoms in the aryl moiety, and
Az is 1-triazolyl optionally substituted by halogen or methyl, or a metal complex thereof, for combating unwanted plant growth.

2. A compound of the formula II

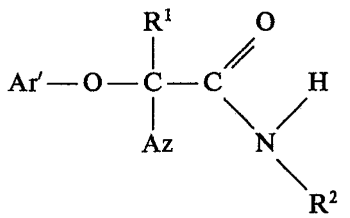

(II)

where

Ar' is 3,5-dichlorophenyl or 3,5-dimethylphenyl,
$R^1$ is hydrogen or methyl,
$R^2$ is alkyl of 1 to 10 carbon atoms, cycloalkyl of 3 to 8 carbon atoms, cyclopropylmethyl, allyl, methallyl, crotyl, 2-methylbut-2-en-1-yl, 2-methylbut-1-en-3-yl, hex-5-en-1-yl, 2-methylbut-1-en-4-yl, propargyl, but-1-yn-3-yl, but-2-yn-1-yl, 3-methylbut-1-yn-3-yl or an optionally halogen-substituted benzyl, and
Az is 1-triazolyl optionally substituted by halogen or methyl, or a metal complex thereof.

3. A compound als claimed in claim 2, namely 2-(3,5-dichlorophenoxy)-2-(1,2,4-triazol-1-yl)-acetic acid-N-tert-butylamide or 2-(3,5-dichlorophenoxy)-2-(1,2,4-triazol-1-yl)-acetic acid-N-benzylamide.
4. A herbicide containing a compound as claimed in claim 2.
5. A herbicide containing a compound as claimed in claim 3.
6. The use as claimed in claim 1, wherein a compound as claimed in claim 2 is used.
7. The use as claimed in claim 1, wherein a compound as claimed in claim 3 is used.

**Claims for the Contracting state: AT**

1. The use of a compound of the formula I

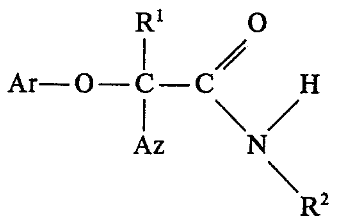

(I)

where

Ar is naphthyl optionally substituted by halogen or phenyl optionally substituted by halogen, alkyl of 1 to 4 carbon atoms, phenyl, benzyl, $CF_3$ or methoxy,
$R^1$ is hydrogen or methyl,
$R^2$ is alkyl of 1 to 10 carbon atoms, cycloalkyl of 3 to 8 carbon atoms, cyclopropylmethyl, allyl, mthallyl, crotyl, 2-methylbut-2-en-1-yl, 2-methylbut-1-en-3-yl, hex-5-en-1-yl, 2-methylbut-1-en-4-yl, propargyl, but-1-yn-3-yl, but-2-yn-1-yl, 3-methylbut-1-yn-3-yl or an optionally halogen-substituted aralkyl having 1 or 2 carbon atoms in the alkyl moiety and 6 to 10 carbon atoms in the aryl moiety, and
Az is triazolyl optionally substituted by halogen or methyl, or a meal complex thereof, for combating unwanted plant growth.

2. A herbicide containing a compound of the formula II

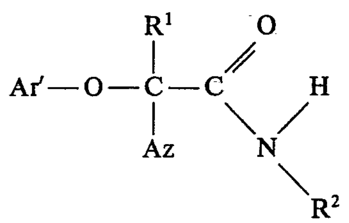

(II)

where

Ar' is 3,5-dichlorophenyl or 3,5-dimethylphenyl,
$R^1$ is hydrogen or methyl,
$R^2$ is alkyl of 1 to 10 carbon atoms, cycloalkyl of 3 to 8 carbon atoms, cyclopropylmethyl, allyl, methallyl, crotyl, 2-methylbut-2-en-1-yl, 2-methylbut-1-en-3-yl, hex-5-en-1-yl, 2-methylbut-1-en-4-yl, propargyl, but-1-yn-3-yl, but-2-yn-1-yl, 3-methylbut-1-yn-3-yl or an optionally halogen-substituted benzyl, and
Az is 1-triazolyl optionally substituted by halogen or methyl, or a metal complex thereof.

3. A herbicide as claimed in claim 2, containing 2-(3,5-diclorophenoxy)-2-(1,2,4-triazol-1-yl)-acetic acid-N-tert-butylamide or 2-(3,5-dichlorophenoxy)-2-(1,2,4-triazol-1-yl)-acetic acid-N-benzylamide.

4. The use as claimed in claim 1, wherein a compound as defined in claim 2 is used.

5. The use as claimed in claim 1, wherein a compound as defined in claim 3 is used.

**Revendications pour les Etats contractants: BE, CH, DE, FR, GB, IT, LI, LU, NL, SE**

1. Utilisation d'un composé de formule I

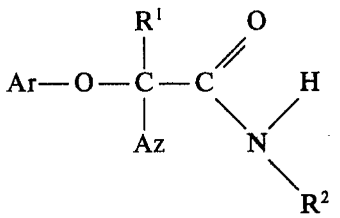

(I)

dans laquelle

Ar représente un reste naphthyle, éventuellement substitué par halogène, ou un reste phényle, éventuellement substitué par halogène, alkyle en $C_1-C_4$, phényle, benzyle, $CF_3$, méthoxy,
$R^1$ représente hydrogène ou méthyle,
$R^2$ un reste alkyle en $C_1-C_{10}$, cycloalkyle en $C_3-C_8$, cyclopropylméthyle, allyle, méthallyle, crotyle, 2-Méthyl-butèn-2-yle-1, 2-méthylbutèn-1-yle-3, hexèn-5-yle-1, 2-méthylbutèn-1-yle-4, propargyle, butin-1-yle-3, butin-2-yle-1, 3-méthyl-butin-1-yle-3, ou un reste aralkyle à 1 à 2 atomes C dans la partie alkyle et 6 à 10 atomes C dans la partie aryle, eventuellement substitué par halogène,
Az représente un reste 1-triazolyle, éventuellement substitué par halogène ou méthyle, ou ses complexes métalliques, pour lutter contre la croissance indésirable des plantes.

2. Composé de formule II

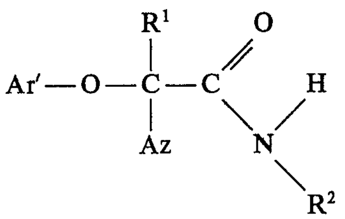

(II)

dans laquelle

Ar' représente un reste 3,5-dichlorophényle ou 3,5-diméthylphényle
$R^1$ représente hydrogène ou méthyle
$R^2$ un reste alkyle en $c_1-C_{10}$, cycloalkyle en $C_3-C_8$, cyclopropylméthyle, allyle, méthallyle, crotyle, 2-méthyl-butèn-2-yle-1, 2-méthylbutèn-1-yle,3, hexèn-5-yle-1, 2-méthylbutèn-1-yle-4, propargyle, butin-1-yle-3, butin-2-yle-1, 3-méthyl-butin-1-yle-3, ou un reste benzyle éventuellement substitué par halogène,
Az représente un reste 1-triazolyle éventuellement substitué par halogène ou méthyle, ou ses complexes métalliques.

3. Composés selon la revendication 2, à savoir: N-tertiaire butylamide d'acide 2-(3,5-dichlorophénoxy)-2-(1,2,4-triazolyl-1) acétique, N-benzylamide d'acide 2-(3,5-dichlorophénoxy)-2-(1,2,4-triazolyle-1) acétique.
4. Herbicide contenant un composé selon la revendication 2.
5. Herbicide contenant un composé selon la revendication 3.
6. Utilisation selon la revendication 1, caractérisée par le fait que l'on utilise un composé selon la revendication 2.
7. Utilisation selon la revendication 1, caractérisée par le fait que l'on utilise un composé selon la revendication 3.

**Revendications pour L'Etat contractant: AT**

1. Utilisation d'un composé de formule I

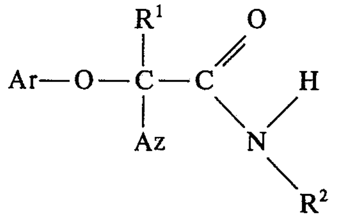

(I)

dans laquelle

Ar représente un reste naphthyle, éventuellement substitué par halogène, ou un reste phényle, éventuellement substitué par halogène, alkyle en $C_1-C_4$, phényle, benzyle, $CF_3$, méthoxy,
$R^1$ représente hydrogène ou méthyle,
$R^2$ un reste alkyle en $C_1-C_{10}$, cycloalkyle en $C_3-C_8$, cyclopropylméthyle, allyle, méthallyle, crotyle, 2-méthyl-butèn-2-yle-1, 2-méthylbutèn-1-yle,3, hexèn-5-yle-1,.2-méthylbutèn-1-yle-4, propargyle, butin-1-yle-3, butin-2-yle-1, 3-méthyl-butin-1-yle-3, ou un reste aralkyle à 1 à 2 atomes C dans la partie alkyle et 6 à 10 atomes C dans la partie aryle, éventuellement substitué par halogène,
Az représente un reste 1-triazolyle éventuellement substitué par halogène ou méthyle, ou ses complexes métalliques, pour lutter contre la croissance indésirable des plantes.

2. Herbicide contenant un composé de formule II

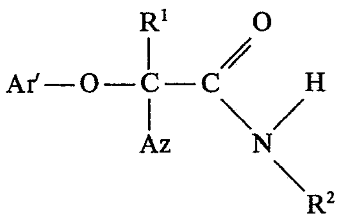

(II)

dans laquelle

Ar' représente un reste 3,5-dichlorophényle ou 3,5-diméthylphényle
$R^1$ représente hydrogène ou méthyle
$R^2$ un reste alkyle en $C_1-C_{10}$, cycloalkyle en $C_3-C_8$, cyclopropylméthyle, allyle, méthallyle, crotyle, 2-méthyl-butèn-2-yle-1, 2-méthylbutèn-1-yle-3, hexèn-5-yle-1, 2-méthylbutèn-1-yle-4, propargyle, butin-1-yle-3, butin-2-yle-1, 3-méthyl-butin-1-yle-3, ou un reste benzyle éventuellement substitué par halogène,
Az représente un reste 1-triazolyle éventuellement substitué par halogène ou méthyle, ou ses complexes métalliques.

3. Herbicide selon la revendication 2 contenant N-tertiaire butylamide d'acide 2-(3,5-dichlorophénoxy)-2-(1,2,4-triazolyl-1) acétique ou N-benzylamide d'acide 2-(3,5-dichlorophénoxy)-2-(1,2,4-triazolyle-1) acétique.

4. Utilisation selon la revendication 1, caractérisée par le fait que l'on utilise un ccomposé selon la revendication 2.

5. Utilisation selon la revendication 1, caractérisée par le fait que l'on utilise un composé selon la revendication 3.